# EUROPEAN PATENT APPLICATION

(11) **EP 2 687 848 A1**
(43) Date of publication of application: **22.01.2014**
(21) Application number: 12176506.9
(22) Date of filing: 16.07.2012
(51) Int. Cl.: G01N 33/569

(54) **Status of tuberculosis infection in an individual**

(71) Applicant: Lionex GmbH, 38126 Braunschweig (DE)
(72) Inventor: Singh, Mahavir, 38124 Braunschweig (DE); Delios, Mario M., 38126 Braunschweig (DE); Della Bella, Chiara, 38126 Braunschweig (DE)
(74) Representative: Kröncke, Rolf

(57) **Abstract**

In a first aspect, the present invention relates to a method for diagnosing or determining the status of tuberculosis infection in an individual afflicted with or suspected to be afflicted with tuberculosis infection. In a further aspect, a method for the stratification of the therapeutic regimen of an individual with tuberculosis infection is provided as well as a method for predicting a clinical outcome or determining treatment course in an individual afflicted with tuberculosis infection. Moreover, the present invention provides a method for monitoring the change from latent into active status of tuberculosis infection or vice versa in an individual. Furthermore, the present invention relates to a kit for use in diagnosing or detecting the status of tuberculosis infection as well as to *Mycobacterium tuberculosis* alanine dehydrogenase for use in specifically differentiating latent status from active diseases status of tuberculosis in an individual.

SEQUENCE LISTING

<110>  Lionex GmbH

<120>  Status of tuberculosis infection in an individual

<130>  3441-11

<160>  1

<170>  PatentIn version 3.5

<210>  1
<211>  371
<212>  PRT
<213>  Mycobacterium tuberculosis

<400>  1

Met Arg Val Gly Ile Pro Thr Glu Thr Lys Asn Asn Glu Phe Arg Val
1               5                   10                  15

Ala Ile Thr Pro Ala Gly Val Ala Glu Leu Thr Arg Arg Gly His Glu
            20                  25                  30

Val Leu Ile Gln Ala Gly Ala Gly Glu Gly Ser Ala Ile Thr Asp Ala
        35                  40                  45

Asp Phe Lys Ala Ala Gly Ala Gln Leu Val Gly Thr Ala Asp Gln Val
    50                  55                  60

Trp Ala Asp Ala Asp Leu Leu Leu Lys Val Lys Glu Pro Ile Ala Ala
65                  70                  75                  80

Glu Tyr Gly Arg Leu Arg His Gly Gln Ile Leu Phe Thr Phe Leu His
                85                  90                  95

Leu Ala Ala Ser Arg Ala Cys Thr Asp Ala Leu Leu Asp Ser Gly Thr
            100                 105                 110

Thr Ser Ile Ala Tyr Glu Thr Val Gln Thr Ala Asp Gly Ala Leu Pro
        115                 120                 125

Leu Leu Ala Pro Met Ser Glu Val Ala Gly Arg Leu Ala Ala Gln Val
    130                 135                 140

Gly Ala Tyr His Leu Met Arg Thr Gln Gly Gly Arg Gly Val Leu Met
145                 150                 155                 160

Gly Gly Val Pro Gly Val Glu Pro Ala Asp Val Val Val Ile Gly Ala
                165                 170                 175

Gly Thr Ala Gly Tyr Asn Ala Ala Arg Ile Ala Asn Gly Met Gly Ala
            180                 185                 190

Thr Val Thr Val Leu Asp Ile Asn Ile Asp Lys Leu Arg Gln Leu Asp

195                 200                 205

Ala Glu Phe Cys Gly Arg Ile His Thr Arg Tyr Ser Ser Ala Tyr Glu
    210                 215                 220

Leu Glu Gly Ala Val Lys Arg Ala Asp Leu Val Ile Gly Ala Val Leu
225                 230                 235                 240

Val Pro Gly Ala Lys Ala Pro Lys Leu Val Ser Asn Ser Leu Val Ala
                245                 250                 255

His Met Lys Pro Gly Ala Val Leu Val Asp Ile Ala Ile Asp Gln Gly
            260                 265                 270

Gly Cys Phe Glu Gly Ser Arg Pro Thr Thr Tyr Asp His Pro Thr Phe
        275                 280                 285

Ala Val His Asp Thr Leu Phe Tyr Cys Val Ala Asn Met Pro Ala Ser
    290                 295                 300

Val Pro Lys Thr Ser Thr Tyr Ala Leu Thr Asn Ala Thr Met Pro Tyr
305                 310                 315                 320

Val Leu Glu Leu Ala Asp His Gly Trp Arg Ala Ala Cys Arg Ser Asn
                325                 330                 335

Pro Ala Leu Ala Lys Gly Leu Ser Thr His Glu Gly Ala Leu Leu Ser
            340                 345                 350

Glu Arg Val Ala Thr Asp Leu Gly Val Pro Phe Thr Glu Pro Ala Ser
        355                 360                 365

Val Leu Ala
    370

## Description

In a first aspect, the present invention relates to a method for diagnosing or determining the status of tuberculosis infection in an individual afflicted with or suspected to be afflicted with tuberculosis infection. In a further aspect, a method for the stratification of the therapeutic regimen of an individual with tuberculosis infection is provided as well as a method for predicting a clinical outcome or determining treatment course in an individual afflicted with tuberculosis infection. Moreover, the present invention provides a method for monitoring the change from latent into active status of tuberculosis infection or vice versa in an individual. Furthermore, the present invention relates to a kit for use in diagnosing or detecting the status of tuberculosis infection as well as to *Mycobacterium tuberculosis* alanine dehydrogenase for use in specifically differentiating latent status from active diseases status of tuberculosis in an individual.

### Prior art

Over the past decade substantial advances have been achieved in the in vitro immunological diagnosis of *Mycobacterium tuberculosis* (TB) infection (Machingaidze S. et al., Pediatr Infect Dis J. 2011; 30:694-700). Three *Mycobacterium tuberculosis* specific interferon-γ release assays (IGRAs) are commercially available. They detect interferon-γ (INF-γ) release by mononuclear cells in the ELISpot (T-SPOT. *TB* - Oxford Immunotec, Abingdon, UK) or in whole blood in the ELISA (QuantiFERON-TB GOLD and QuantiFERON-TB GOLD in tube; QFT-G-IT; Cellestis, Carnegie, Australia) following *ex vivo* contact with specific *Mycobacterium tuberculosis* antigens. With respect of tuberculin skin test (TST), IGRAs have a number of advantages: they are minimally influenced by previous Bacille Calmette-Guérin (BCG) vaccination or infection by non-tuberculosis mycobacteria, do not cause booster effect , do not necessitate a double access to health care facility , and interpretation of results is not operator-dependent (Chiappini E. et al., Clin Ther. 2012 Apr 16). In adults IGRAs have been reported to be more specific and at least as sensitive as TST and are currently included in diagnostic algorithms in adult guidelines (Mazurek GH. et al., United States, 2010, MMWR Recomm Rep. 2010; 59:1-25.). However, reported IGRAs sensitivity and specificity largely vary among studies in paediatric populations and caution is recommended regarding their use in children because of poor and contrasting data (Machingaidze S. et. al., Pediatr Infect Dis J. 201 1 ;30:694-700; Sun L. et. al., FEMS Immunol. Med. Microbiol. 2011 ;63:165-73; Mandalakas AM, et. al., Int J Tuberc Lung Dis 2011;15:1018-32). Moreover, they do not allow to distinguish active from latent tuberculosis infection (LTBI) (Amanatidou V. et. al., Eur J Clin Microbiol Infect Dis. 2012 Jan 4). This issue is fundamental for paediatricians since a definite TB diagnosis, confirmed by cultures, is rare in children. Paediatric TB disease is typically paucibacillary and most TB diagnoses in children are "probable "diseases, based on TST and IGRA results, clinical symptoms and signs, radiological findings, personal history, response to TB therapy, and physicians' experience in this field.

A positive IGRA in a child with signs suggestive of active TB does not allow the definitive diagnosis especially if the child comes from a high TB prevalence area. The positive IGRA result may be due to TB disease as well as LTBI, which may be incidental to the disease causing the symptoms or signs under investigation. In this situation, a test that accurately distinguishes active tuberculosis from latent TB infection would be valuable. Although results have been achieved in studies using ELISpot assays based on cytokines other than IFN-γ (i.e.:IL-10 or IL-2) (Amanatidou V. et. al., Eur J. Clin Microbiol Infect Dis. 2012 Jan 4) or exploring immune response to other mycobacterial antigens besides ESAT-6, CFP-10 and TB7.7 which are currently included in the commercially available IGRAs.

Hence, there is a need for further methods allowing to differentiate between latent and active status of tuberculosis infection. Accordingly, there is a need in the art for approaches that are for detection and determination of the therapeutic regimen or, predicting the clinical outcome or determining the treatment course as well as determining the status of tuberculosis infection in an individual suffering therefrom.

The present invention aims for providing new methods and assays particularly useful in the issues described above.

### Summary of the present invention

In a first aspect, the present invention relates to a method for diagnosing or determining the status of tuberculosis infection in an individual afflicted with or suspected to be afflicted with tuberculosis infection, comprising:
a) determining the level or amount of cytokines released or produced by mononuclear cells after stimulation, whereby said mononuclear cells are obtained from said individual; and
b) determining or diagnosing the status of said individual based on the level or amount of cytokines released or produced from the mononuclear cells,
characterized in that the mononuclear cells are stimulated with mycobacterial Alanine Dehydrogenase (AlaDH).

In a further aspect, the present invention relates to a method for the stratification of the therapeutic regimen of an individual with tuberculosis infection comprising:
a) determining the level or amount of cytokines released or produced from mononuclear cells after stimulation, whereby said mononuclear cells are obtained from said individual; and
b) determining the status of tuberculosis infection based on the level or amount of cytokines released or produced from said mononuclear cells after stimulation allowing differentiation of a latent and active status of tuberculosis infection in said individual, characterized in that the mononuclear cells are stimulated in the presence of mycobacterial Alanine Dehydrogenase (AlaDH), in particular, of *Mycobacterium tuberculosis.*

In another aspect, the present invention relates to a method for predicting a clinical outcome or determining the treatment course in an individual afflicted with tuberculosis infection comprising:
a) determining the level or amount of cytokines released or produced from mononuclear cells after stimulation, whereby said mononuclear cells are obtained from said individual; and
b) predicting the clinical outcome or determining the treatment course based on the level or amount of cytokines released or produced from said mononuclear cells after stimulation, characterized in that the stimulation includes cultivation of said mononuclear cells with mycobacterial Alanine Dehydrogenase (AlaDH), in particular, of *Mycobacterium tuberculosis.*

Another embodiment of the present invention relates to a method for monitoring the change from latent to active status of tuberculosis infection or vice versa in an individual comprising:
a) determining the level or amount of cytokines released or produced from mononuclear cells from said individual after stimulation at a first point in time;
b) determining the level or amount of cytokines released or produced from mononuclear cells after stimulation, whereby said mononuclear cells are obtained from said individual at a second point in time; and
c) comparing the level or amount of cytokines determined in step a) to the level or amount determined in step b) or to a reference value whereby an increase in the level or the amount relative to a reference value or to the level or amount determined in step a) is indicative for a transition from latent to active status and a decrease in the level or the amount relative to a reference value or to the level or amount determined in step a) is indicative for a transition from active to latent status,
characterized in that the mononuclear cells are stimulated with mycobacterial Alanine Dehydrogenase (AlaDH), in particular, of *Mycobacterium tuberculosis.*

That is, the present inventors recognised that the mycobacterial, in particular, the *Mycobacterium tuberculosis* Alanine Dehydrogenase (AlaDH) represents a suitable stimulant for mononuclear cells obtained from the individuals afflicted with or suspected to be afflicted with tuberculosis infection and, subsequently, determining the amount or level of cytokines released or produced by said mononuclear cells after stimulation, thus, allowing to differentiate between latent and active status of tuberculosis infection in said individual, accordingly. In particular, contrary to other known mycobacterial components, e.g. as described in Amanatidou V. et. al., Eur J Clin Microbiol Infect Dis. 2012 Jan 4, it has been shown surprisingly that AlaDH results in cytokine release or production in active TB state only while during latent TB state cytokine release or production is significantly lower. This is particularly true for the cytokine IL-2.

Moreover, the present invention relates to the use of a kit for diagnosing or determining the status of tuberculosis infection, or for predicting a clinical outcome or determining the treatment course in an individual afflicted with or suspected to be afflicted with tuberculosis infection, or for the stratification of the therapeutic regimen of an individual with tuberculosis infection or for monitoring the progression of tuberculosis infection, or for determining the transition from latent to active status of tuberculosis infection in an individual, said kit comprises means for determining the level or amount of cytokines released or produced from mononuclear cells after stimulation, whereby said mononuclear cells are obtained from said individual, a stimulating agent which is mycobacterial, e.g. of *Mycobacterium tuberculosis,* AlaDH and instructions on how to use said test kit for a method according to the present invention. Further, the present invention relates to the use of mycobacterial Alanine Dehydrogenase, in particular, of *Mycobacterium tuberculosis,* as a stimulant allowing to differentiate between latent and active status of tuberculosis infection in a individual afflicted with or suspected to be afflicted with tuberculosis infection.

Finally, a composition comprising mycobacterial AlaDH, in particular, of *Mycobacterium tuberculosis,* in combination ESAT-6 and CFP-10 is provided for use in determining tuberculosis infection in an individual.

### Brief description of the drawings

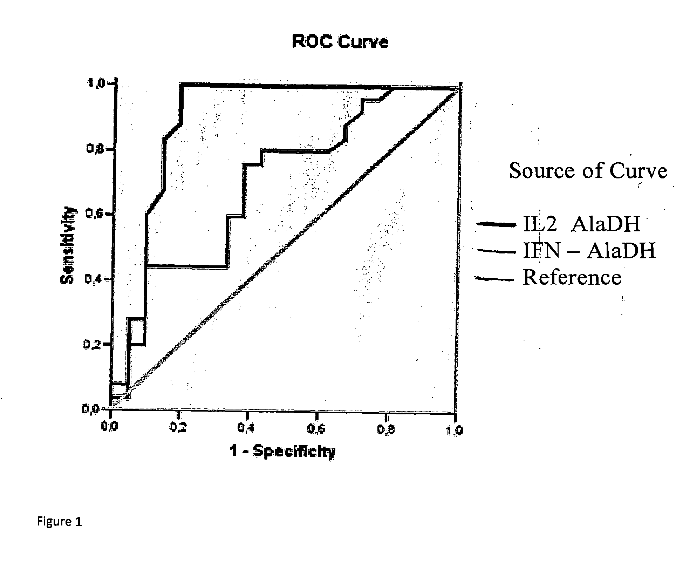

Figure 1 : Figure 2 shows ROC illustrating sensitivity and specificity of AlaDH IFN-gamma and IL-2 ELISpot results in discriminating children with latent (n = 21) and active (n = 25) tuberculosis.

### Detailed description of the present invention

The present invention relates in a first aspect to a method for diagnosing or determining the status of tuberculosis infection in an individual afflicted with or suspected to be afflicted with tuberculosis infection, comprising:
a) determining the level or amount of cytokines released or produced by mononuclear cells after stimulation, whereby said mononuclear cells are obtained from said individual; and
b) determining or diagnosing the status of said individual based on the level or amount of cytokines released or produced from the mononuclear cells,
characterized in that the mononuclear cells are stimulated with mycobacterial Alanine Dehydrogenase (AlaDH), in particular, of *Mycobacterium tuberculosis.* That is, the present inventors recognised that depending on the level or amount of cytokines released or produced by mononuclear cells after stimulation whereby said mononuclear cells are obtained from the individual afflicted with or suspected to be effected with tuberculosis infection, it is possible to diagnose or determine the status of tuberculosis infection in said individual when nuclear cells are stimulated with mycobacterial Alanine Dehydrogenase, in particular, of *Mycobacterium tuberculosis,* for cytokine release or production.

In contrast to assays commercially available, the assays and methods described herein allows to differentiate between the latent and active status of tuberculosis infection. The differentiation between the two different types of status is important for determining the treatment course and the risk of the course of tuberculosis infection as well as for predicting the clinical outcome. Moreover, it is important to monitor the development of the tuberculosis infection in said individual. It has been shown that when using AlaDH as stimulant, the cytokine pattern released or produced from the mononuclear cells stimulated with the AlaDH molecule is different between individuals having latent TB infection and individuals having active TB infection. In contrast, the molecules of commercially available IGRAS allow to differentiate between non-infected and infected individuals only without differentiating between the two status.

As used herein, the term "individual" in the context of the present invention is typically a mammal, in particular, the mammal is a human, a non-human primate or other mammals susceptible for mycobacterial infection. The individual can be male or female. The individual can be one who has been previously diagnosed with or identified as suffering from tuberculosis infection and, optionally, but need not to have already undergone treatment for tuberculosis infection. An individual can also be one who has been diagnosed with or identified as suffering from tuberculosis infection but who has shown improvement in the disease as a result of receiving one or more treatments for the infection, accordingly. Moreover, the individual may also be one who has not been previously diagnosed or identified as having tuberculosis infection.

The term "determining" or "detecting" as used herein refers to assessing the presence, absence, quantity, level or amount of either a given substance within a clinical or subject derived sample, including quality and/or quantitative concentration levels of substances otherwise evaluating values or categorisation of a subject's clinical parameter.

The term "comprises" or "comprising" or "contains" or "containing" includes the embodiments of "consist" or "consisting".

As used herein, the term "AlaDH" comprises antigenic fragments or the whole polypeptide of AlaDH. In particular, the AlaDH is an antigenic oligopeptide or polypeptide derived from the peptide of Seq ID No. 1. That is, the mycobacterial AlaDH is preferably the ALaDH of TB with the NCBI-reference No. NP_217296.1 or the TubercuList database No. AlaDH-Rv2780. The skilled person is well aware of suitable fragments, e.g. as described in EP 0966544 being incorporated herein by reference. In addition, the term comprises homologs thereof of Seq. ID. No. 1 having the same stimulatory activity on mononuclear cells as AlaDH of Seq. ID. No. 1.

As used herein, the term "latent" includes the latent status of TB infection (LTBI) as well as the cured status after successful treatment unless otherwise indicated.

Moreover, the present invention relates to method for the stratification of the therapeutic regimen of an individual with tuberculosis infection comprising:
a) determining the level or amount of cytokines released or produced from mononuclear cells after stimulation, whereby said mononuclear cells are obtained from said individual; and
b) determining the status of tuberculosis infection based on the level or amount of cytokines released or produced from said mononuclear cells after stimulation allowing differentiation of a latent and active status of tuberculosis infection in said individual, characterized in that the mononuclear cells are stimulated in the presence of mycobacterial Alanine Dehydrogenase (AlaDH), in particular, of *Mycobacterium tuberculosis.*

That is, it is possible to stratify the therapeutic regimen of an individual suffering from tuberculosis infection based on determining the status of infection, namely to differentiate between latent and active status of tuberculosis infection.

Further, the present inventors recognised that AlaDH represents a valuable tool, namely, a valuable stimulant for mononuclear cells obtained from an individual afflicted with or suspected to be afflicted with tuberculosis infection for diagnosing or determining the status of said tuberculosis infection in said individual.

Hence, a method is provided for monitoring the change from latent to active status of tuberculosis infection or vice versa in an individual comprising:
a) determining the level or amount of cytokines released or produced from mononuclear cells from said individual after stimulation at a first point in time;
b) determining the level or amount of cytokines released or produced from mononuclear cells after stimulation, whereby said mononuclear cells are obtained from said individual at a second point in time; and
c) comparing the level or amount of cytokines determined in step a) to the level or amount determined in step b) or to a reference value whereby an increase in the level or the amount relative to a reference value or to the level or amount determined in step a) is indicative for a transition from latent to active status and an decrease in the level or the amount relative to a reference value or to the level or amount determined in step a) is indicative for a transition from active to latent status
characterized in that the mononuclear cells are stimulated with mycobacterial Alanine Dehydrogenase (AlaDH), in particular, of *Mycobacterium tuberculosis.*

In the context of the present invention, the term "reference value" refers to an index value, a value derived from one or more computer indices, a value derived from a subject with known active or latent tuberculosis infection, in particular, with a cohort of the same. In particular, the reference values obtained from individuals afflicted with latent and active status tuberculosis infection and, in addition, the reference value represents a range or index obtained from at least two samples collected from individual of said disease state.

In a preferred embodiment, the mononuclear cells are obtained from a sample of said individual. Said sample may be blood, a blood derived sample or other samples containing mononuclear cells suitable for allowing cytokine release assays or assays determining the amount of cytokines both on protein and nucleic acid level. In particular, the sample is a blood sample or is derived from other body fluids including Bronchoalveolar lavage and urine and tissues.

In particular, the mononuclear cells are obtained from blood and are isolated by common techniques.

A person skilled in the art will appreciate a number of different methods are useful for obtaining said mononuclear cells from the individual.

It is preferred that the cytokine level or amount is determined by immuno based assays. A person skilled in the art will appreciate that a number of methods are useful for determining the amount or level of the cytokine in a sample, including immunoassays such as western blot, ELISA and ELIspot. It is particularly desired that the cytokines are determined at protein level with ELIspot or ELISA technique. However, the cytokine levels can also be measured by determining the mRNA levels corresponding to the said cytokines, accordingly. The skilled person is well aware of suitable methods for determining the same on nucleic acid level including PCR techniques.

It is preferred that the cytokine determined is at least one selected from IL-2 or interferon-γ. It is particularly desired that the IL-2 amount or level is determined released or produced by said mononuclear cells after stimulation with mycobacterial Alanine Dehydrogenase (AlaDH), in particular, of *Mycobacterium tuberculosis.*

As demonstrated herein, IL-2 ELIspot based ELIspot assays allow to differentiate between latent and active status of tuberculosis infections in either children or adults.

According to a preferred embodiment, the reference value for the latent status of tuberculosis infection is below 25, preferably below 10, while the reference value for the active status of tuberculosis infection is above 50, preferably above 100, for IL-2 ELIspot.

When monitoring the change from latent to active status of tuberculosis infection or vice versa in an individual, the first sample from the individual is preferably obtained at a first time point while the second sample obtained after a, preferably predetermined, time point, e.g. after undergoing treatment of tuberculosis infection or undergoing treatment for autoimmune diseases. For example in case of the treatment of autoimmune diseases or other diseases whereby the immune system is modulated, it is helpful to determine the status of TB infection to avoid any onset of the TB infection. It is known that when treatment RA patents, latent status TB infection may proceed to active status. That is, the present invention allows to determine the treatment course in said individual or predicting the clinical outcome by identifying the actual status of tuberculosis infection in said individual.

In particular, the method according to the present invention allows for identifying or monitoring or supervising individuals afflicted with tuberculosis infection to determine a change from latent to active status of tuberculosis or vice versa. When the relative or absolute level or amount of cytokines increases, it is indicative for a transition from the latent to active status while a decrease in the relative or absolute level or amount is indicative for a transition from active to latent status.

In clear contrast to mycobacterial Alanine Dehydrogenase (AlaDH), in particular, of *Mycobacterium tuberculosis,* other stimulants used e.g. in ELlspot or ELISA assays as stimulants allow to differentiate between TB infection and non TB infection only, e.g. the well known mycobacterial molecules ESAT-6, CFP-10 or other molecules.

However, it has been determined herein that a composition comprising mycobacterial Alanine Dehydrogenase (AlaDH), in particular, of *Mycobacterium tuberculosis,* and ESAT-6 as well as CFP-10 allows to increase the sensitivity and specificity of tests allowing to differentiate between uninfected and infected individuals.

Important clinical implications arise from the finding described herein. Due to the possibility to differentiate between latent and active status it is possible to stratify the therapeutic regimen or to determine the treatment course in the individual accordingly.

In a further aspect, the present invention relates to the use of a kit for diagnosing or determining the status of tuberculosis infection, or for predicting a clinical outcome or determining the treatment course in an individual afflicted with or suspected to be afflicted with tuberculosis infection, or for the stratification of the therapeutic regiment of an individual with tuberculosis infection or for monitoring the progression of tuberculosis infection, or for determining the transition from latent to active status of tuberculosis infection in an individual, said kit comprises means for determining the level or amount of cytokines released or produced from mononuclear cells after stimulation, whereby said mononuclear cells are obtained from said individual, a stimulating agent which is mycobacterial Alanine Dehydrogenase (AlaDH), in particular, of *Mycobacterium tuberculosis,* and instructions on how to use said test kit for a method according to the present invention.

In particular, in a preferred embodiment said kit is an ELIspot or ELISA allowing for determining the level or amount of the cytokine. It is particular preferred that the ELIspot or ELISA is an assay allowing determination of the amount or level of IL-2 or interferon-γ, in particular, IL-2. Alternatively, the kit is a PCR kit with means suitable for analysing cytokine expression on nucleic acid level.

Moreover, the present invention relates to the use of mycobacterial Alanine Dehydrogenase (AlaDH), in particular, of *Mycobacterium tuberculosis,* in differentiating latent status and active status of tuberculosis infection in an individual afflict with or suspected to be afflicted with tuberculosis infection. The mycobacteria Alanine Dehydrogenase is particularly useful for differentiating the same in human, in particular, children.

Finally, the present invention provides assays useful for performing or conducting the method according to the present invention. Said assay comprises means for culturing mononuclear cells and AlaDH as stimulant in a form suitable to stimulate said mononuclear cells when added to a culture thereof. Furthermore, the assay may contain means for determining the amount or level of the cytokine to be detected. Typically, said assay is based on an immunoassay, in particular, said assay is an EliSpot or ELISA. Detection is effected by antibodies detecting specifically the desired cytokine. Alternatively, the assay is a PCR-based assay.

The above disclosure generally describes the present invention. More complete understanding can be obtained by reference to the following specific examples of third embodiments of the invention without being limited thereto.

### Examples

### Materials and Methods

### Study subjects

Consecutive children at risk for TB infection referred to the Infectious Disease Unit, Department of Department of Sciences for Woman and Child's Health University of Florence, Italy, were prospectively enrolled between 2009 and 2010. Study children were those with clinical suspicion of TB disease and/or in close contact with recently diagnosed cases of contagious TB disease and/or internationally adopted or recently immigrated children coming from countries with a high-prevalence of TB. A maximum of two years was considered as period of recent immigration. Children with congenital or acquired immunodeficiency disorders (based on their medical history, clinical examination and/or laboratory tests) were excluded from the study.

### Tuberculin skin test

TST was administered according to the Mantoux method by injecting intradermally 5 tuberculin units (in 0.1 mL) of purified protein derivative (Biocine Test-PPD, Chiron, Siena, Italy) into the volar surface of the forearm. The transversed skin induration was recorded (in mm) after 48-72 hours. Following the American Academy of Pediatrics guidelines (American Academy of Pediatrics. [Tuberculosis]. In: Pickering LK, Baker CJ, Kimberlin DW, Long SS, eds. Red Book: 2009 Report of the Committee on Infectious Diseases. 28th ed. Elk Grove Village, IL: American Academy of Pediatrics; 2009:[680-701]. Amanatidou V. et. al, see above) a positive TST was defined as an induration size ≥ 5 mm for children in close contact with known or suspected contagious case of TB disease or for children suspected to have TB disease (based on clinical evidence and/or chest radiograph) and ≥ 10 mm for children born in countries with a high prevalence of TB and recently immigrated.

### QFT-G-IT

The QFT-IT test was performed according to the manufacturer's instructions as described previously (Millington KA, et. al., Proc Natl Acad Sci USA.2011 ;108:5730-5. Vilaplana C, et. al., Scand J Immunol 2008;67:610-7). After subtracting the value from the negative control, the result was positive if, the antigen-dependent response was ≥0.35 IU, negative if the mitogen-induced response was ≥0.5 IU/mL and the antigen-dependent response was <0.35 IU/mL, and indeterminate if both mitogen-induced and antigen-dependent responses were below cut-off or mitogen-induced response >8 IU/mL.

### ELISpot Assay

In-house ELIspot for IFN-gamma and IL-2 were performed using Lionex M. tuberculosis. antigens. Briefly, T cell blasts (105 cells) of each patients line were stimulated with M. tuberculosis antigen in the presence of irradiated autologous APCs (5 × 104 cells) and seeded in triplicate in 96-well plates coated with anti-IFN-γ or anti-IL-2 antibody. Cells stimulated with medium alone served as negative controls. Cells stimulated with PHA served as positive controls. IFN-γ and IL-2 ELISPOT microplates were then incubated at 37°C in 5% CO2 for 24 hours. At the end of the culture period, plates were washed and incubated for 3 hours with the appropriate biotinylated anti-IFN-γ or anti-IL-2 mAb. Streptavidin-HRP complex was then added for 2 hours, followed by the substrate solution. SFCs were counted using an automated ELISPOT reader (Autoimmune Diagnostika GmbH).

### Study design

Information regarding socio-demographic data, prior TB exposure, and past medical history was obtained from each child's parents or tutors or from medical documentation and recorded into the study database. Children were considered vaccinated with BCG whether a clear documentation was available and/or a scar was present. All children underwent clinical evaluation, TST and venipuncture for IGRAs (QFT-G-IT and in-house ELISpot assay). Blood was taken during the first examination after the parent's or tutor's informed consent had been obtained and before starting any anti-tubercular treatment. Chest radiography was performed in all symptomatic children, in those with a positive TST, and in all contacts aged less than 5 years. Children with suspected pulmonary TB had three sputum or early morning gastric aspirates samples collected for Mycobacterium tuberculosis detection (by means of microscopy, polymerase chain reaction and culture). Chest computed tomography (CT) scan was performed in selected cases at the paediatrician's discretion. No suspected or ascertained extra-pulmonary TB cases presented at the Infectious Disease Unit during the study period. The study received local Ethical Committee approval and informed consent for the study was obtained by parents or tutors.

### Definition of study groups

Study children were classified as not-infected, LTBI cases, or active lung TB disease cases, following the American Academy Guidelines definition (American Academy of Pediatrics. [Tuberculosis]. In: Pickering LK, Baker CJ, Kimberlin DW, Long SS. eds. Red Book: 2009 Report of the Committee on Infectious Diseases. 28th ed. Elk Grove Village, IL: American Academy of Pediatrics: 2009:[680-701]). In the event of discordant TST/QFT-G-IT results, children were assigned to the corresponding group on the bases on the TST result, according to the most recent U.S. and European Guidelines (American Academy of Pediatrics. [Tuberculosis]. In: Pickering LK, Baker CJ, Kimberlin DW, Long SS. eds. Red Book: 2009 Report of the Committee on Infectious Diseases. 28th ed. Elk Grove Village, IL: American Academy of Pediatrics: 2009:[680-701]. Amanatidou V. et. al., see above). In particular, asymptomatic children with negative TST were defined as uninfected. LTBI diagnosis was assigned to any child with a positive TST and no clinical nor radiographic evidence of active TB. Cases of active TB were defined according to two categories: definite TB, children with *Mycobacterium* tuberculosis cultured or detected by microscopy or molecular methods from sputum or gastric aspirate culture, and probable TB: absence of microbiological confirmation but presence of all of the following criteria: (A) clinical symptoms and signs of active TB, (B) abnormal radiography and/or CT scan consistent with lung TB, (C) response to TB therapy plus, (D) either a history of TB contact or travel to a TB-endemic country within the last 24 months (Amanatidou V. et. al, see above).

### Statistical analysis

Categorical data were compared using the Chi-squared test (or Fisher's exact test, when expected cell sizes were smaller than five). The Wilcoxon-Mann-Whitney test was used for continuous measurements to test relationships in unpaired analysis, when assumed that the dependent variable is a not normally distributed interval variable. Test concordance was assessed by Cohen's κ-statistics with agreement considered slight' for k≤0.2, 'fair' for 0.2<k≤0.4, 'moderate' for 0.4<k≤0.6, 'substantial' for 0.6<k≤0.8 and 'optimal' for 0.8<k≤1.0. Receiver operating characteristic (ROC) curve analysis was conducted to determine the best IL-2 and IFN-γ ELISpot result thresholds in discriminating between children with active or latent TB, relatively to specific M. tuberculosis antigens and correspondent sensitivity and specificity were reported. The area under the ROC curve (AUC) and 95% confidence interval (CI) were also calculated. Statistical analysis was performed using the statistical software SPSS for Windows, version 14.0. P <0.05 was considered statistically significant.

### Results

Seventy five children (males); median age: 75 months; interquartile range [IQR] 39-116) were included in the study. Previous BCG vaccination was documented in 21 (28.0%) children.

The diagnosis of definite TB disease was made in 5 children. All these children presented with symptoms compatible with TB (e.g. persistent cough, fever, night sweats or weight loss) and/or pulmonary disease documented by chest X-ray and confirmed by CT scan results and *Mycobacterium* tuberculosis was detected from the cultures and/or microscopy and/or polymerase chain reaction. Probable TB was diagnosed in 20 children. All these children underwent at least three gastric aspirate or sputum examination for *Mycobacterium* tuberculosis detection which yielded negative results. Table 1 shows the BCG vaccination status, the TST size and the QFT-G-IT result according to final diagnosis.

**Table 1: BCG vaccination status, TST size and QFT- G-IT result according to final diagnosis of the study children**

| | | uninfected | latent TB | Probable TB disease | Definite TB disease |
|---|---|---|---|---|---|
| | | n = 29 | n = 21 | n = 20 | n = 5 |
| | | n (%) | n (%) | n (%) | n (%) |
| Age, months (median and IQR) | | 77 (28-101) | 116 (61-151) | 52(27-82) | 47 (8-97) |
| BCG vaccinated TST, induration diameter (mm) | | 5 | 12 | 1 | 0 |
| | < 5 | 28 | 0 | 0 | 1 |
| | ≥ 5 and < 10 | 1 | 1 | 2 | 0 |
| | ≥ 10 and < 15 | 0 | 5 | 1 | 1 |
| | ≥ 15 | 0 | 15 | 17 | 3 |

| QFT-G-IT result | | | | | |
|---|---|---|---|---|---|
| | negative | 29 | 5 | 3 | 5 |
| | positive | 0 | 15 | 17 | 0 |
| | indeterminate | 0 | 1 | 0 | 0 |

| | | | | | |
|---|---|---|---|---|---|
| BCG = Bacille Calmette-Guérin, TST = tuberculin skin test, IGRA = interferon-γ release assay | | | | | |

Discordant TST/QFT-G-IT result was obtained in four children (one child with definite TB, positive QFT-G-IT and negative TST and three children with probable TB, negative QFT-G-IT and positive TST). The overall agreement between the IGRA and the TST (without considering the indeterminate result) was substantial with a k value of 0.679.

### IFN-γ and IL-2 ELISpot results

### ELISpot results are summarized in Table 2.

**Table 2. ELISpot results**

| | uninfected | latent TB | Probable TB disease | Definite TB disease | P Uninfected vs. infected children | P Latent vs. overt disease |
|---|---|---|---|---|---|---|
| | n = 29 | n = 21 | n = 20 | n = 5 | | |
| IFN-γ ELISpot | | | | | | |
| - ESAT-6 | 35 (1-102) | 320 (157-520) | 292 (170-631) | 330 (190-1575) | <0.0001 | 0.724 |
| - CFP-10 | 40 (1-120) | 305 (130-625) | 280 (76-986) | 875 (200-3532) | <0.0001 | 0.691 |
| - H1-hybrid | 60 (12-192) | 300 (127-437) | 295 (120-825) | 180 (115-1737) | <0.0001 | 0.651 |
| - Ag85B | 65 (12-152) | 150 (47-322) | 107 (46-192) | 90 (27-1517) | 0.063 | 0.724 |
| - AlaDH | 35 (12.5-110) | 85 (17-180) | 147 (71-303) | 280 (115-1565) | <0.001 | 0.021 |
| - PstS1 | 40 (1-122) | 50 (1-197) | 60 (11-241) | 95 (18-982) | 0.0512 | 0.504 |

| IL-2 ELISpot | | | | | | |
|---|---|---|---|---|---|---|
| - ESAT-6 | 10 (1-35) | 220 (35-410) | 110 (60-402) | 420 (155-1365) | <0.0001 | 0.791 |
| - CFP-10 | 10 (1-20) | 210 (85-710) | 160 (100-637) | 510 (355-1735) | <0.0001 | 0.574 |
| - H1-hybrid | 20 (1-50) | 180 (35-535) | 195 (95-595) | 430 (265-1635) | <0.0001 | 0.247 |
| - Ag85B | 20 (10-75) | 40 (10-140) | 45 (17-195) | 200 (135-1485) | 0.063 | 0.188 |
| - AlaDH | 1 (1-20) | 1 (1-10) | 75 (36-130) | 340 (110-1365) | =0.001 | <0.0001 |
| - PstS1 | 10 (1-50) | 20 (1-75) | 20 (10-95) | 10 (5-155) | <0.0001 | 0.755 |

Comparing uninfected children vs. children with TB infection (LTBI plus probable and definite TB disease) significantly higher median values were evidenced in infected than in uninfected children considering IFN-γ ELISpot responses to ESAT-6 (p<0.0001), CFP-10 (p<0.0001), H1-hybrid (p<0.0001) or AlaDH (p=0.001) while differences were not significant considering Ag85B (p=0.063) and Pst1S (p=0.512). Considering IL-2 ELISpot results significantly different results were evidenced with ESAT-6, CFP-10, H1 hybrid, and AlaDH (p=0.001); but not with Ag85 (p=0.063).

Comparing results between children with LTBI and those with TB disease (probable plus definite disease) differences were significant for IFN-γ ELISpot only considering response to AlaDH antigen (p=0.021) (Table 2). With regard to IL-2 ELISpot significant differences were confirmed for AlaDH antigen (p<0.0001) while no difference was evidenced considering other antigens. Comparing results between uninfected children and children with LTBI significant difference were observed in IFN-γ ELISpot responses to ESAT-6 (p>0.0001), CFP-10(p>0.0001), H1-hybrid (p=0.001), Mix (p=0.017) and in IL-2 ELISpot responses to ESAT-6 (p>0.0001), CFP-10 (p>0.0001), H1-hybrid (p>0.0001), and mix (p>0.0001).

ROC analyses demonstrated that, considering response to AlaDH, for IL-2 ELISpot value of 12.5 SCF per million PBMC (after the subtraction of *background spots)* sensitivity in discriminating between latent and active TB was 100% and specificity was 81 %. For IFN-γ ELISpot the performance was much poorer: for the best threshold of 42.0 SCF per million PBMC sensitivity was 88% and specificity 56% (Figure 1).

Similar results were obtained in adult LTBI patients, see table 3.

**Table 3. Adult ELISpot results**

| | uninfected | latent TB | Probable TB disease | Definite TB disease |
|---|---|---|---|---|
| | n = 31 | n = 39 | n=12 | n = 18 |
| IFN-γ ELISpot | | | | |
| - ESAT-6 | 29 | 343 | 301 | 342 |
| - CFP-10 0 | 34 | 298 | 276 | 722 |
| - H1-hybrid | 52 | 312 | 221 | 173 |
| - Ag85B | 71 | 179 | 114 | 102 |
| - AlaDH | 24 | 99 | 266 | 328 |
| - PstS1 | 51 | 43 | 72 | 86 |

| IL-2 ELISpot | | | | |
|---|---|---|---|---|
| - ESAT-6 | 7 | 215 | 114 | 433 |
| - CFP-10 0 | 5 | 227 | 148 | 471 |
| - H1-hybrid | 14 | 198 | 201 | 445 |
| - Ag85B | 25 | 52 | 63 | 217 |
| - AlaDH | 2 | 8 | 193 | 429 |
| - PstS1 | 9 | 29 | 37 | 13 |

As demonstrated by way of examples, the performances of interferon-γ and IL-2 based ELIspot assays in children and adult when using *Mycobacterium tuberculosis* AlaDH as stimulant (antigen) allows to differentiate between latent and acute status of tuberculosis infection. Moreover, as expected, the antigens currently included in commercial available IGRAs like ESAT-6 and CFP-10 allows to differentiate between uninfected and infected individuals only.

Moreover a mixture or composition of mycobacterial *Mycobacterium tuberculosis* AlaDH and ESAT-6 as well as CFP-1 0 allows to determine tuberculosis infection in an individual with higher sensitivity and specificity. As demonstrated by ROC-analysis, sensitivity of discriminating a latent and active status reached 100 % while specificity was 96 % by the use of IL-2 based ELIspot assay. The performance of the interferon-γ ELIspot assay using AlaDH antigen was not as good as the IL-2 based ELIspot assay but still allow to differentiate between the two different types of status.

Moreover, as demonstrated, the methods and assay systems according to the present invention are particularly useful for discrimination of children with active from those with latent TB when using AlaDH antigen.

## Claims

1. A method for diagnosing or determining the status of tuberculosis infection in an individual afflicted with or suspected to be afflicted with tuberculosis infection, comprising:
a) determining the level or amount of cytokines released or produced by mononuclear cells after stimulation, whereby said mononuclear cells are obtained from said individual; and
b) determining or diagnosing the status of said individual based on the level or amount of cytokines released or produced from the mononuclear cells,
**characterized in that** the mononuclear cells are stimulated with with mycobacterial Alanine Dehydrogenase (AlaDH), in particular, of *Mycobacterium tuberculosis.*

2. A method for the stratification of the therapeutic regimen of an individual with tuberculosis infection comprising:
a) determining the level or amount of cytokines released or produced from mononuclear cells after stimulation, whereby said mononuclear cells are obtained from said individual; and
b) determining the status of tuberculosis infection based on the level or amount of cytokines released or produced from said mononuclear cells after stimulation allowing differentiation of a latent and active status of tuberculosis infection in said individual, **characterized in that** the mononuclear cells are stimulated in the presence of with mycobacterial Alanine Dehydrogenase (AlaDH), in particular, of *Mycobacterium tuberculosis.*

3. A method for predicting a clinical outcome or determining the treatment course in an individual afflicted with tuberculosis infection comprising:
a) determining the level or amount of cytokines released or produced from mononuclear cells after stimulation, whereby said mononuclear cells are obtained from said individual; and
b) predicting the clinical outcome or determining the treatment course based on the level or amount of cytokines released or produced from said mononuclear cells after stimulation, **characterized in that** the stimulation includes cultivation of said mononuclear cells with mycobacterial Alanine Dehydrogenase (AlaDH), in particular, of *Mycobacterium tuberculosis.*

4. A method for monitoring the change from latent to active status of tuberculosis infection or vice versa in an individual comprising:
a) determining the level or amount of cytokines released or produced from mononuclear cells from said individual after stimulation at a first point in time;
b) determining the level or amount of cytokines released or produced from mononuclear cells after stimulation, whereby said mononuclear cells are obtained from said individual at a second point in time; and
c) comparing the level or amount of cytokines determined in step a) to the level or amount determined in step b) or to a reference value whereby an increase in the level or the amount relative to a reference value or to the level or amount determined in step a) is indicative for a transition from latent to active status and a decrease in the level or the amount relative to a reference value or to the level or amount determined in step a) is indicative for a transition from active to latent status
**characterized in that** the mononuclear cells are stimulated with mycobacterial Alanine Dehydrogenase (AlaDH), in particular, of *Mycobacterium tuberculosis.*

5. The method according to any one of the preceding claims wherein the mononuclear cells are stemming from a sample of the individual including a blood sample or other body fluids including Bronchoalveolar lavage and urine and tissues.

6. The method according to any one of the preceding claims wherein the level or amount of cytokines is determined at protein level, in particular, by an immunoassay, like, ELISpot or ELISA or at nucleic acid level, in particular, by PCR techniques.

7. The method according to any one of the preceding claims wherein the cytokines to be determined are selected from IL-2 and interferon Gamma, in particular, IL-2.

8. The method according to any one of the preceding claims wherein the reference value in IL-2 Elispot is below 25.

9. The method according to any one of the preceding claims wherein the individuals are children.

10. The use of a kit for diagnosing or determining the status of tuberculosis infection, or for predicting a clinical outcome or determining the treatment course in an individual afflicted with or suspected to be afflicted with tuberculosis infection, or for the stratification of the therapeutic regiment of an individual with tuberculosis infection or for monitoring the progression of tuberculosis infection, or for determining the transition from latent to active status of tuberculosis infection in an individual, said kit comprises means for determining the level or amount of cytokines released or produced from mononuclear cells after stimulation, whereby said mononuclear cells are obtained from said individual, a stimulating agent which is mycobacterial Alanine Dehydrogenase (AlaDH), in particular, of *Mycobacterium tuberculosis,* and instructions on how to use said test kit for a method according to anyone of claims 1 to 9.

11. The use of a kit according to claim 10 whereby said kit is an ELISpot or ELISA or said kit is a PCR kit.

12. Mycobacterial Alanine Dehydrogenase, in particular, of *Mycobacterium tuberculosis,* for use in differentiating latent status and active status of tuberculosis infection in an individual afflicted with or suspected to be afflicted with tuberculosis infection.

13. The mycobacterial Alanine Dehydrogenase (AlaDH), in particular, of *Mycobacterium tuberculosis,* for use in differentiating between latent and active tuberculosis infection according to claim 12 wherein the individuals are children.

14. An assay for conducting the method according to any one of claims 1 to 9 comprising means for culturing mononuclear cells, means for determining the level or amount of cytokines and mycobacterial Alanine Dehydrogenase (AlaDH), in particular, of *Mycobacterium tuberculosis.*

15. Composition comprising mycobacterial Alanine Dehydrogenase (AlaDH), in particular, of *Mycobacterium tuberculosis* and ESAT-6 and CFP-1 0 for use in determining tuberculosis infection in an individual.
